# EUROPÄISCHE PATENTSCHRIFT

(11) **EP 2 379 725 B1**
(45) Veröffentlichungstag und Bekanntmachung des Hinweises auf die Patenterteilung: **27.04.2016**
(21) Anmeldenummer: 09810742.8
(22) Anmeldetag: 18.12.2009
(51) Int. Cl.: C12N 15/82, C12N 9/26

(54) **VERFAHREN ZUR STEIGERUNG DES SACCHAROSEERTRAGES BEIM LANDWIRTSCHAFTLICHEN ANBAU VON ZUCKERRÜBEN UND ZUCKERROHR**
METHOD FOR INCREASING THE SACCHAROSE YIELD IN AGRICULTURAL PRODUCTION OF SUGAR BEETS AND SUGAR CANE
PROCÉDÉ D'AUGMENTATION DU RENDEMENT DE SACCHAROSE DANS LA CULTURE DE LA BETTERAVE SUCRIÈRE ET DE LA CANNE À SUCRE

(30) Priorität: 22.12.2008 DE 102008064184
(43) Veröffentlichungstag der Anmeldung: 26.10.2011
(73) Patentinhaber: KWS SAAT SE, 37574 Einbeck (DE); Südzucker AG Mannheim/Ochsenfurt, 68165 Mannheim (DE)
(72) Erfinder: HARMS, Karsten, 67549 Worms (DE); SCHULZ, Britta, 37574 Einbeck (DE)
(74) Vertreter: Westhoff, Markus
(86) Internationale Anmeldenummer: PCT/DE2009/001797
(87) Internationale Veröffentlichungsnummer: WO 2010/072210

(56) Entgegenhaltungen:
- WO-A1-98/04722
- WO-A2-02/50109
- TANG GUO-QING ET AL: "Antisense repression of vacuolar and cell wall invertase in transgenic carrot alters early plant development and sucrose partitioning" PLANT CELL, AMERICAN SOCIETY OF PLANT PHYSIOLOGISTS, ROCKVILLE, MD, US, Bd. 11, Nr. 2, 1. Februar 1999 (1999-02-01), Seiten 177-189, XP002444994 ISSN: 1040-4651
- RAUSCH T & GREINER S: "Plant protein inhibitors of invertases" BIOCHIMICA ET BIOPHYSICA ACTA (BBA) - PROTEINS & PROTEOMICS, ELSEVIER LNKD- DOI:10.1016/J.BBAPAP.2003.09.017, Bd. 1696, Nr. 2, 12. Februar 2004 (2004-02-12), Seiten 253-261, XP004489014 ISSN: 1570-9639
- GODT D & ROITSCH T: "The developmental and organ specific expression of sucrose cleaving enzymes in sugar beet suggests a transition between apoplasmic and symplasmic phloem unloading in the tap roots" PLANT PHYSIOLOGY AND BIOCHEMISTRY, GAUTHIER-VILLARS, PARIS, FR LNKD- DOI:10.1016/J.PLAPHY.2006.09.019, Bd. 44, Nr. 11-12, 19. Dezember 2006 (2006-12-19), Seiten 656-665, XP005808168 ISSN: 0981-9428
- ROHWER J M & BOTHA F C: "Analysis of sucrose accumulation in the sugar cane culm on the basis of in vitro kinetic data" BIOCHEMICAL JOURNAL, Bd. 358, Nr. 2, 1. September 2001 (2001-09-01), Seiten 437-445, XP9133462 ISSN: 0264-6021

### SEQUENZPROTOKOLL

<110> KWS SAAT AG
<120> Verfahren zum landwirtschaftlichen Anbau von Zuckerrüben
<130> KWS 0157
<160> 2
<170> PatentIn version 3.3
<210> 1
   <211> 811
   <212> DNA
   <213> Nicotiana tabacum
<400> 1
   cggcacgaga acaaaaccaa acacctttcc tttggcctct cctcctttta tcttttatat     60
   caatcctcat cttcaataac accactctca aaacaaatga gaaacttatt ccccatattt    120
   atgttaatca ccaatctagc attcaacgac aacaacaaca gtaataatat catcaacacg    180
   acctgcagag ccaccacaaa ctaccccttg tgcctcacca ccctccactc tgatccccgt    240
   acctccgagg ccgagggggc ggacctcacc accctcggcc tcgtcatggt agatgcggta    300
   aaattaaagt ccatcgaaat aatgaaaagt ataaaaaaac tcgaaaaatc gaaccccgag    360
   ttgagactac ctcttagcca atgttacata gtgtattatg ctgttctaca tgctgatgta    420
   actgttgctg ttgaagcttt aaaaagagga gtccctaaat ttgctgaaaa tggaatggtt    480
   gatgttgctg tagaagcaga aacttgtgag tttagtttta agtataatgg attggtttct    540
   ccagtttctg atatgaataa ggagattatt gaactgtctt ctgtggctaa atctattatt    600
   agaatgctat tatgaggaaa ttaaagaacc aaagatacaa ggttctggtt atgttagttt    660
   attagtgctg taataggatt tttatattcc tgtgtttttt ttgctttttt tatttcattt    720
   gggtgcttgt gtgtatatgt gaaaatgagt gtgaattatg tcaaacataa acatagatta    780
   gaaattactc ctgaaaaaaa aaaaaaaaaa a                                   811
<210> 2
   <211> 555
   <212> DNA
   <213> Beta vulgaris
<400> 2
   atgacaactc taaacacctc tttaccacat cttcacctcc tcttcattac ccttcttaca     60
   ctattcacca cctctacttt agcgtattct cgcaagacca ccaacgacct tgtaaccacc    120
   acgtgcaagc aaacacccga cccgattctt tgcgaagctt cgctccgatc agactctcgg    180
   agctccaagg ctgctgactc tgaaggttta atcctgatca tgatcgacgt tgtcaaaact    240
   cggttttcgg actcgtttcg atatgtagag gacttgaccc ggaagaccca tgacccggat    300
   gtaatccggg ccctgcaaga gtgtaagcaa ctctatcggg ttgtgttaga tgtaagtgta    360
   ggtttagcag tgagagcagt aaagcaaggg gatccgaaat tcggggagca agctatggtg    420
   gatgcgggta atgaggccga ggggtgtcgg atggcgttcc cggaaggtaa ggttccgggt    480
   cggatcgtgg gtcgaacacg gatgctccat ggagtatcta atgtggctgc ttctatgatt    540
   aagagtttgg aatga                                                     555

## Beschreibung

Die vorliegende Erfindung betrifft die Verwendung einer Nukleinsäure, die in einer Pflanzenzelle zur Reduzierung der enzymatischen Aktivität einer Invertase geeignet ist sowie die Verwendung von Zuckerrüben- oder Zuckerrohrpflanzen zur Steigerung des Saccharoseertrages beim landwirtschaftlichen Anbau von Zuckerrüben und Zuckerrohr.

Zuckerrübenpflanzen sind zweijährige Pflanzen. Im ersten Jahr der Entwicklung erfolgt das vegetative Wachstum, wobei die Pflanze eine Blattrosette entwickelt und eine Primärwurzel, den Rübenkörper, ausbildet. Im zweiten Jahr, der generativen Phase, werden Blütenstand und Samen ausgebildet. Die Besonderheit der Ertragsphysiologie der Zuckerrübe liegt darin, dass die Akkumulation und Speicherung von Saccharose überwiegend im ersten Vegetationsjahr erfolgt. Für die Saccharosegewinnung werden Zuckerrüben deshalb im vegetativen Entwicklungsstadium geerntet.

Jahrzehntelange Bemühungen und Erfolge in der Zuckerrübenzüchtung haben beachtenswerte Zuwächse hinsichtlich des Rübenkörper- und Saccharoseertrags gebracht. Allerdings sind diese Zuwächse noch immer nicht zufriedenstellend, obgleich die Saccharosekonzentration des Rübenkörpers heute bereits ca, 15-20 % des Rübenkörperfrischgewichts beträgt.

Aus EP 0 956 357 B1 ist die Verwendung einer Nukleinsäure bekannt, die für ein zur Reduzierung der enzymatischen Aktivität einer Invertase befähigtes Polypeptid kodiert, zur Herstellung einer transgenen Pflanze mit reduziertem, lagerungsbedingten Saccharose-Verlust. Einen Hinweis auf die Möglichkeit, mit Hilfe eines Invertaseinhibitors die Saccharosekonzentration in einem Saccharosespeicherorgan einer Pflanze zu steigern, gibt diese Druckschrift nicht.

Darüber hinaus sieht EP 1 346 054 B1 die Hemmung einer pflanzlichen vacuolären Invertase vor. Auch diese Druckschrift beschreibt lediglich eine verbesserte Lagerstabilität von Pflanzen und keine Erhöhung der Saccharosekonzentration ,

Aufgabe der vorliegenden Erfindung ist es daher, die Saccharosekonzentration in einem Saccharosespeicherorgan einer Pflanze weiter zu steigern. Insbesondere besteht die Aufgabe der vorliegenden Erfindung darin, den landwirtschaftlichen Anbau von Zuckerrüben und Zuckerrohr zu verbessern, indem der Saccharoseertrag gesteigert wird.

Erfindungsgemäß erfolgt die Lösung der gestellten Aufgabe durch die Verwendung einer Nukleinsäure, die in einer Pflanzenzelle zur Reduzierung der enzymatischen Aktivität einer Invertase geeignet ist, zur Ausbildung eines Saccharosespeicherorgans einer Pflanze, bei dem die Saccharosekonzentration gegenüber der Saccharosekonzentration eines nicht veränderten Kontrollsaccharosespeicherorgans des gleichen Genotyps in vergleichbarem Entwicklungsstadium erhöht ist.

Bislang sind Saccharosekonzentration und Rübenkörperertrag jedoch negativ korreliert, d. h. Sorten mit hoher Saccharosekonzentration liefern einen geringeren Rübenkörperertrag als Sorten mit niedrigerer Saccharosekonzentration. Der Grund hierfür liegt darin, dass Zuckerrüben mit einer hohen Saccharosekonzentration einen eher kleinen Rübenkörper ausbilden.

Ein Erklärungsversuch für die negative Beziehung zwischen Saccharosekonzentration und Rübenkörperertrag bei Zuckerrüben wurden bereits 1973 von Milford unternommen. Danach sind Rübengewicht und die Größe der parenchymatischen Speicherzellen korreliert. Große Parenchymzellen weisen demnach eine geringere Saccharosekonzentration als kleine Parenchymzellen auf.

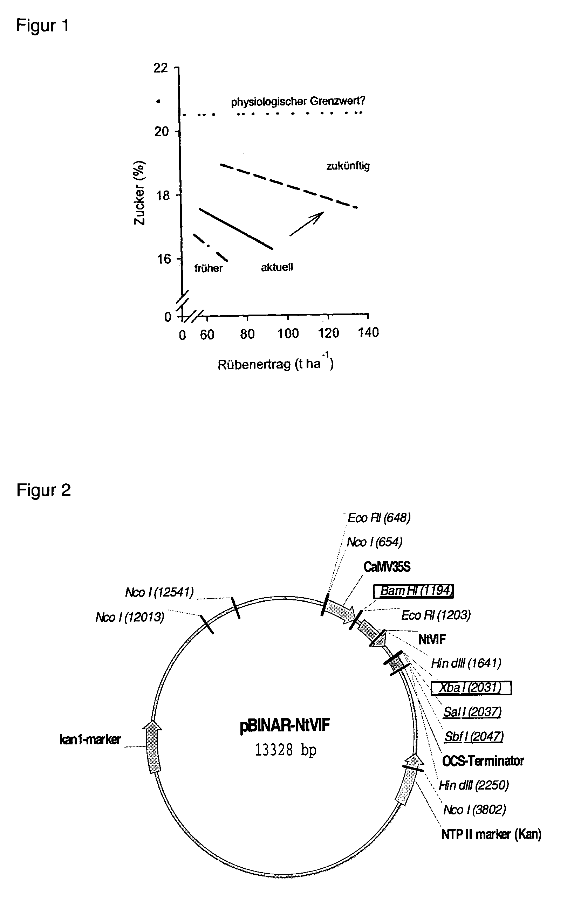

Die Beziehung zwischen Saccharosekonzentration und Rübenkörperertrag ist auch bei neuen Sorten negativ, allerdings nicht mehr so eng wie es früher beschrieben wurde (Campbell und Kern 1983, Hoffmann und Märländer 2002). Der Züchtungsfortschritt führte somit nur zu einer Verschiebung der negativen Beziehung zwischen Saccharosekonzentration und Rübenkörperertrag, nicht jedoch zu einer grundlegenden Änderung dieser Beziehung (Figur 1).

Die oben für Zuckerrüben beschriebene negative Beziehung zwischen Saccharosekonzentration und Saccharosespeicherorganertrag gilt grundsätzlich auch für Zuckerrohr.

In überraschender Weise wurde jetzt gefunden, dass bei der erfindungsgemäßen Verwendung der Nukleinsäure, die in einer Pflanzenzelle zur Reduzierung der enzymatischen Aktivität einer Invertase geeignet ist, auch die negative Korrelation zwischen Saccharosekonzentration und Saccharosespeicherorganertrag (Zuckerrübenkörperertrag bzw. Zuckerrohrhalmertrag) zurückgeführt bzw. aufgehoben werden kann.

Insbesondere kann die erfindungsgemäße Verwendung der Nukleinsäure bei einer Erhöhung der Saccharosekonzentration um X Prozentpunkte zu einem verringerten Saccharosespeicherorganertrag führen, wobei die Verringerung des Saccharosespeicherorganertrags maximal 5X Prozent beträgt.

Nach einer besonderen Ausgestaltung der Erfindung ist bei der Verwendung der Nukleinsäure, die in einer Pflanzenzelle zur Reduzierung der enzymatischen Aktivität einer Invertase geeignet ist, der Saccharosespeicherorganertrag daher unverändert oder erhöht.

So führt die Erhöhung der Saccharosekonzentration um beispielsweise 2 Prozentpunkte im Falle einer Verringerung des Saccharosespeicherorganertrags zu einer Verringerung von maximal 10 Prozent. In der Regel führt die Erhöhung der Saccharosekonzentration jedoch auch zu einer Erhöhung des Saccharosespeicherorganertrags, so dass die Saccharosekonzentration des Saccharosespeicherorgans nicht negativ korreliert ist mit dem Saccharosepeicherorganertrag.

Im Falle der Zuckerrübe gibt die Saccharosekonzentration die Menge an Saccharose in der Rübenkörperfrischmasse an, und zwar in Prozent. Unter Rübenkörperertrag wird die Frischmasse von erdfreien, optimal geköpften Rüben verstanden. Aus dem Rübenkörperertrag multipliziert mit der Saccharosekonzentration errechnet sich dann der Saccharoseertrag.

Ebenfalls am Beispiel der Zuckerrübe wird der Vorteil der vorliegenden Erfindung anhand einer unten aufgeführten Beispielberechnung verdeutlicht.

Als besonders geeignete Nukleinsäure zur Reduzierung der enzymatischen Aktivität einer Invertase dient eine Nukleinsäure, die für einen Invertaseinhibitor codiert. Invertaseinhibitoren wurden erstmals in Kartoffel beschrieben (Schwimmer et al. 1961). Sie sind aus Zuckerrübe, Tomate und Tabak bekannt (Pressey 1968, Pressey 1994, Weil et al. 1994, Rausch und Greiner 2004). Doch obwohl Invertaseinhibitoren zum Stand der Technik gehören, war es bislang nicht zu erwarten, dass mittels dieser Polypeptide die Saccharosekonzentration eines Zuckerrübenkörpers gesteigert werden kann. Insbesondere war es nicht bekannt, hierdurch auch die negative Beziehung zwischen Saccharosekonzentration und Saccharosespeicherorganertrag zurückzuführen.

Andererseits stellt die Verfügbarkeit verschiedener Inveraseinhibitoren auch eine gute Voraussetzung für die neue Verwendung dar. So kann die Nukleinsäure, die für ein zur Reduzierung der enzymatischen Aktivität einer Invertase befähigtes Polypeptid kodiert, problemlos identifiziert bzw. erhalten und bei der Zuckerrübe und beim Zuckerrohr eingesetzt werden, und zwar in züchterischer Weise ebenso wie mittels gentechnischer Methoden.

Eine andere Möglichkeit zur Reduzierung der enzymatischen Aktivität einer Invertase lässt sich aber auch dadurch erreichen, dass man die enzymatische Aktivität einer Invertase nicht über ein inhibierendes Polypeptid, sondern beispielsweise mittels Antisense-RNA oder RNA-Interferenz reduziert. Beide Verfahren gehören mittlerweile zu allgemein bekannten Standardtechniken. Im Falle der Antisense-RNA wird eine einzelsträngige RNA eingesetzt, die komplementär zur m-RNA der Invertase ist. Durch anschließende Paarung der komplementären RNAs kommt es zu einer Inhibierung der Translation der m-RNA und damit zur Verhinderung der Genexpression der Invertase in der Zelle. Bei der RNA-Interferenz nutzt man dagegen einen Inverted Repeat eines cDNA-Fragmentes, welcher nach Transkription ein doppelsträngiges RNA-Molekül bildet, das von pflanzeneigenen Enzymen in Fragmente mit einer Länge von ungefähr 21 bis 23 Nukleotiden gespalten wird, die zu einer Translationsblockade der m-RNA der Invertase führen.

Invertasen aus Zuckerrübe sind ebenfalls bekannt (Rosenkranz et al., 2001, Gonzales et al., 2005) bzw. lassen sich anhand bekannter Invertasen (Sturm und Chrispeels, 1990; Sturm, 1999) aus Zuckerrübe und Zuckerrohr ohne besondere Schwierigkeit isolieren, so dass auch entsprechende Antisense-RNA- oder RNA-Interferenz-Konstrukte ohne Weiteres herstellbar sind. Mittels allgemein bekannter Pflanzentransformationsverfahren lassen sich solche Konstrukte gut in Pflanzen übertragen und dort exprimieren.

Nach einer weiter bevorzugten Ausgestaltung der Erfindung handelt es sich bei der verwendeten Nukleinsäure um eine heterologe Nukleinsäure, die aus *Nicotiana tabacum* stammt.

Der Begriff "heterolog" bedeutet, dass die betreffende Nukleinsäure in Bezug auf die Wirtszelle einen anderen Ursprung hat oder zumindest in einem nicht natürlichen Zustand vorliegt. Wird eine Wirtszelle mit einer Nukleinsäure transformiert, die aus einem artfremden Organismus stammt, ist die Nukleinsäuresequenz heterolog zu der Wirtszelle und zu den Nachkommen der Wirtszelle, die diese Nukleinsäuresequenz tragen.

In überraschender Weise konnten bei transgenen Ansätzen auch nur in den aus Samen gezogenen transgenen Nachkommen von Primärtransformanten erhöhte Saccharosekonzentrationen in Rübenkörpern nachgewiesen werden. Die Rübengewichte waren gegenüber der nicht transgenen Kontrolle unverändert, so dass auch ein erhöhter Saccharoseertrag resultierte. In Adventivwurzeln, d.h. rübenkörperähnlichen Gebilden, die von den Primärtransformanten direkt ausgebildet werden, war keine Veränderung der Saccharosekonzentration festzustellen.

Nach einer weiter bevorzugten Ausgestaltung der Erfindung weist die heterologe Nukleinsäure eine Sequenz gemäß der SEQ ID NO: 1 oder eine Nukleinsäuresequenz auf, die mit einer komplementären Sequenz zu der Sequenz gemäß der SEQ ID NO: 1 oder Abschnitten davon hybridisieren kann.

Der hier verwendete Begriff "hybridisieren" bedeutet hybridisieren unter üblichen Bedingungen, wie sie in Sambrook et al. (1989) beschrieben sind, bevorzugt unter stringenten Bedingungen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Wenig stringente Hybridisierungsbedingungen sind beispielsweise: Hybridisieren in 4 x SSC bei 37 °C und anschließendes mehrfaches Waschen in 1 x SSC bei Raumtemperatur. "Stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C.

Das von der Nukleinsäure codierte Polypeptid, der Invertaseinhibitor, ist nach erfolgter Transformation in einer Zuckerrüben- oder Zuckerrohrzelle bevorzugt vakuolär, im Cytosol oder in der Zellwand lokalisiert.

Nach einer weiteren Ausgestaltung der Erfindung handelt es sich bei der Nukleinsäure um eine homologe Nukleinsäure. Der Begriff "homolog" bedeutet hierbei, dass die betreffende Nukleinsäure in Bezug auf die Wirtszelle von dem selben natürlichen Ursprung stammt. Der Begriff "homolog" bezieht sich auch auf eine Nukleinsäuresequenz, die in denselben natürlichen ursprünglichen Zelltyp, von dem sie abstammt, eingebracht wird, aber beispielsweise unter Kontrolle eines anderen, in diesem Zelltyp oder in dieser Kombination nicht natürlich vorkommenden, regulatorischen Elementes steht. Dabei sind regulatorische Elemente Sequenzen, die unterstützend an der Expression einer Nukleotidsequenz beteiligt sind. Sie umfassen z.B. Promotoren und Terminatorsequenzen. So kann eine Wirtszelle beispielsweise mit einer Nukleinsäure aus einem artgleichen Organismus transformiert werden, um beispielsweise eine Überexpression dieser Nukleinsäure zu erreichen.

Andererseits ist es möglich, durch bekannte züchterische Maßnahmen die Expression der Nukleinsäure in den Zellen zu erhöhen bzw. dafür zu sorgen, dass die Aktivität des von ihr codierten Polypeptides in den Zellen gesteigert wird. So kann beilspielsweise die allelische Variation am Genort des Invertaseinhibitors aus Zuckerrübe oder Zuckerrohr analysiert werden und dann in der Züchtung mit dem besten Allel weitergearbeitet werden, Grundlage hierfür kann bei Zuckerrübe die Sequenz des Invertaseinhibitors BvC/VIF1 (Beta vulgaris cell wall or vacuolar inhibitor of beta-fructosidase) gemäß der SEQ ID NO: 2 sein sowie eine Nukleinsäuresequenz, die mit einer komplementären Sequenz zu der Sequenz gemäß der SEQ ID NO: 2 oder Abschnitten davon hybridisieren kann.

Die Analyse der allelischen Variation erfolgt in der Weise, dass zunächst durch vergleichende Sequenzierung des Genortes des Invertaseinhibitors die in einer Population vorhandenen Allele identifiziert werden. Dann werden die verschiedenen Allele über wiederholte Rückkreuzungen in immer den gleichen genetischen Hintergrund eingelagert. Die so entstehenden nah-isogenen Linien werden hinsichtlich der Expression des Invertaseinhibitors analysiert. Die Analyse erfolgt mittels eines Northern Blot-Verfahrens oder RT-PCR.

Ein Vergleich der Wirksamkeit der verschiedenen Allele des Invertaseinhibitors kann zudem indirekt über die Enzymaktivität der Invertase gemessen werden.

Nah-isogene Zuckerrübenlinien werden im Feldexperiment angebaut. Nach der Ernte werden Rübenertrag und Saccharosekonzentration bestimmt. Dies erlaubt einen Vergleich der Allele in Bezug auf das Zielmerkmal Saccharoseertrag. Das Allel mit dem besten Effekt bezüglich Saccharoseertrag wird dann in das aktuelle Zuchtmaterial eingekreuzt und zur Sortenentwicklung verwendet.

Die vorliegende Erfindung betrifft auch die Verwendung von Zuckerrüben- oder Zuckerrohrpflanzen mit einer genetischen Ausstattung, die auf die Reduzierung der enzymatischen Aktivität einer Invertase gerichtet ist, zum landwirtschaftlichen Anbau von Zuckerrüben und Zuckerrohr, wobei die genetische Ausstattung die Ausbildung eines Saccharosespeicherorgans ermöglicht, bei dem die Saccharosekonzentration gegenüber der Saccharosekonzentration eines nicht veränderten Kontrollsaccharosespeicherorgans des gleichen Genotyps in vergleichbarem Entwicklungsstadium erhöht ist. Dabei kann die Erhöhung der Saccharosekonzentration um X Prozentpunkte zu einem verringerten Saccharosespeicherorganertrag führen, wobei die Verringerung des Saccharosespeicherorganertrags maximal 5X Prozent beträgt. In bevorzugter Weise ist der Saccharosespeicherorganertrag unverändert oder erhöht.

Die genetische Ausstattung der Zuckerrüben- oder Zuckerrohrpflanzen kann wiederum mittels gentechnischer Methoden oder konventioneller Pflanzenzüchtungsmethoden erfolgen. Insofern wird auf die oben genannten Möglichkeiten zur neuen Verwendung einer Nukleinsäure verwiesen.

Eine bevorzugte Ausgestaltung der erfindungsgemäßen Verwendung sieht vor, dass die genetische Ausstattung die Ausbildung eines Saccharosespeicherorgans ermöglicht, bei dem die Saccharosekonzentration nicht negativ korreliert ist mit dem Saccharosespeicherorganertrag.

Die folgende Berechnung verdeutlicht am Beispiel der Zuckerrübe, wie sich die neue Verwendung auf den Saccharoseertrag insgesamt auswirken kann.

Der Saccharoseertrag einer Standard-Zuckerrübensorte beträgt bei einem Rübenertrag von 60 t/ha und einer Saccharosekonzentration der Rübe von 17% insgesamt 10,2 t/ha. Mit der erfindungsgemäßen Verwendung lassen sich dagegen Saccharosekonzentrationen von 18% und mehr erreichen. Bei gleichem Rübenertrag ergibt eine Konzentrationssteigerung von 1% bereits eine Saccharoseertragssteigerung von 600 kg Saccharose pro Hektar Anbaufläche. Aufgrund der bislang negativen Korrelation zwischen Saccharosekonzentration und Rübenertrag führten bisherige Steigerungen der Saccharosekonzentration von zum Beispiel 17% auf 18% zu einem überproportional verringerten Rübenertrag, so dass eine Konzentrationssteigerung der Saccharosekonzentration nur sehr bedingt sinnvoll war.

Die Erfindung wird nachfolgend anhand der Figuren und eines bevorzugten Ausführungsbeispiels näher erläutert.

Züchtungsfortschritt nimmt zwar die Steigung der Regressionsgeraden weiter ab, die Beziehung zwischen Rübenkörperertrag und Saccharosekonzentration ist aber auch bei neuen Sorten negativ.

Figur 2 zeigt den Vektor, in dem der Invertaseinhibitor aus ***Nicotiana tabacum*** (SEQ ID NO: 1) integriert wurde (pBINAR-NtVIF).

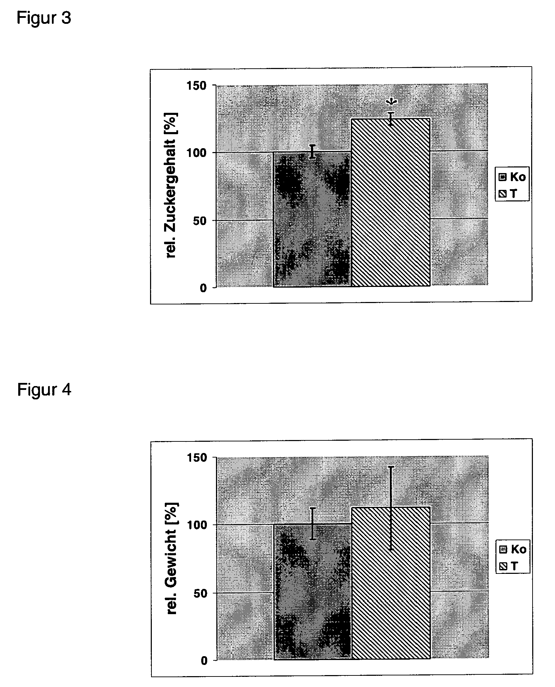

Figur 3 zeigt den relativen Saccharosegehalt in transgenen Linien T (n=25) im Vergleich zu nicht transgenen Kontrollen K₀ (n=40). Sämtliche Pflanzen wurden im Gewächshaus in Röhren angezogen und 204 Tage nach dem Auslegen der Samen geerntet. Die transgenen Linien unterscheiden sich mit einem um 24% höheren Saccharosegehalt signifikant von den nicht transgenen Kontrollen.

Figur 4 zeigt das relative Gewicht der Rübenkörper der Pflanzen nach Figur 3. Das Gewicht der Rübenkörper der transgenen Linien unterscheidet sich in diesem Falle nicht von dem der Kontrollrüben. Damit weisen die transgenen Linien im Vergleich zu nicht transgenen Kontrollen einen erhöhten Saccharosegehalt bei nicht signifikant verändertem Rübengewicht auf. Die negative Beziehung zwischen Saccharosegehalt und Rübenertrag ist in diesem Falle gebrochen.

### Beispiel für die Verwendung einer heterologen Nukleinsäure

Über die Restriktionsschnittstellen *Bam*HI (1194) und *Xba*I (2031) wurde die ausgewählte für einen Invertaseinhibitor aus *Nicotiana tabacum* kodierende cDNA mit einer Länge von 811 bp (SEQ ID NO: 1) in den Vektor pBINAR integriert, so dass Plasmid pBINAR-NtVIF resultierte.

Die Transformation der Zuckerrüben erfolgte nach Lindsey et al. (1991). Die Transgenität der Pflanzen wurde durch PCR überprüft. Nach Regeneration wurden die Transformanten zur Gewinnung von Saatgut im Gewächshaus angezogen und geselbstet.

Das durch Selbstung erhaltene Saatgut der ausgewählten transgenen Linie und der nicht transgenen Kontrolle wurde Anfang März ausgelegt und die Keimlinge Mitte März vereinzelt. Die Anzucht erfolgte im Gewächshaus in Röhren (19 x 100 cm) bei einer Temperatur von ∼16 °C und einer Lichtstärke von 6 Klux und einer Lichtdauer von 18 Stunden. Die Röhren enthielten Topferde FE Typ 340 (Fruhsdorfer), Pikiererde LAT Terra P8 und 5 g Triabon. Wöchentlich wurden die Pflanzen mit Kamasol 3-4 ‰ gedüngt und gegen Mehltau und Läuse behandelt. 204 Tage nach dem Auslegen wurden die Rübenkörper geerntet, geköpft, gewaschen, gewogen und über eine Einzelrübensäge verbreit. Der Brei wurde direkt bei -20 °C eingefroren.

### Saccharose-Bestimmung

26 g des Zuckerrübenbreis wurden mit 177 ml Extraktionslösung (3 g Aluminiumsulfat in 1000 ml Reinstwasser) 5 min. bei Raumtemperatur unter Rühren extrahiert. Die Mischung wurde über Rundfilter 240 mm filtriert und anschließend mit Reinstwasser 1:10 verdünnt. Über Spritzenvorsatzfilter wurden 1,5 ml Verdünnungsfiltrat in Glasvials zur HPLC-Analyse überführt. 10 µl des Verdünnungsfiltrats wurden in einer HP1100 HPLC-Anlage von Agilent Technologie mit einem RI Detektor über eine Merck LichroCart 250-3 Säule (Lichrospher 100 NH₂ (5 µm) Cat.No. 1.50834), in einem Laufmittel von 70 % Acetonitril (v/v Acetonitril 70, Wasser 30) und einer Flussrate von 0,8 ml/min analysiert. Der ermittelte HPLC-Wert gibt Saccharose in µg/ml wieder und wurde unter Einbezug des Verdünnungsfaktors in % Saccharose bezogen auf das Rübenfrischgewicht umgerechnet. Zur Kalibration wurden externe Standardlösungen von 100 µg Saccharose/ml Wasser, 500 µg Saccharose/ml Wasser und 5000 µg Saccharose/ml Wasser verwendet.

### Referenzen

Campbell, L.G. und Kern, J.J. (1983). Relationship among components of yield and quality of sugarbeets. J. Am. Soc. Sugar Beet Technol. 22,135-145.

Gonzales, M.-C., Roitsch, T., Cejudo, F. (2005). Circadian and developmental regulation of vacuolar invertase expression in petioles of sugar beet plants. Planta 222 (2) 386 - 395.

Hoffmann, C. (2006). Zuckerrüben als Rohstoff - Die technische Qualität als Voraussetzung für eine effiziente Verarbeitung. ISBN 978-3-93033-87-5.

Hoffmann, C. und Märländer, B. (2002). Züchterischer Fortschritt in Ertrag und Qualität von Zuckerrüben. Zuckerind. 127, 425-429.

Lindsey, K., Gallois, P., Eady, C. (1991). Regeneration and transformation of sugar beet by Agrobacterium tumefaciens. Plant Tissue Culture Manual B7: 1-13; Kluwer Academic Publisher.

Milford, G.F.J. (1973). The growth and development of the storage root of sugar beet. Ann. Appl. Biol. 75, 427-438.

Pressey, R. (1968). Invertase inhibitor from red beet, sugar beet and sweet potato roots. Plant Physiol., 43, 1430-1434.

Pressey, R. (1994). Invertase inhibitor in tomato fruit. Phytochemistry, 36, 543-546.

Rausch T., Greiner, S. (2004). Plant protein inhibitors of invertases. Biochimica et Biophysica Acta, 1696, 253-261.

Rosenkranz, H., Vogel, M., Greiner, S., Rausch, T. (2001). In wounded sugar beet (Beta vulgaris L.) tap-root, hexose accumulation correlates with the induction of a vacuolar invertase isoform. J. Exp. Bot., 52 (365), 2381 - 2385.

Sambrook, J., Fritsch, E..F., and Maniatis, T (1989). In Molecular Cloning, A Laboratrory Manual (Cold Spring Harbor Laboratory Press, New York).

Schwimmer, S., Makower, R.U., Rorem, E.S. (1961). Invertase and invertase inhibitor in potato. Plant Physiol., 36, 313-316.

Sturm, A., (1999). Invertases. Primary structures, functions, and roles in plant development and sucrose partitioning. Plant Physiology 121, 1-7.

Sturm, A., Chrispeels, M.J. (1990). cDNA cloning of carrot extracellular betafructosidase and ist expression in response to wounding and bacterial infection. Plant Cell 2, 1107 -1119.

Weil, M., Krausgrill, S., Schuster, A., Rausch, T., (1994). A 17-kDa Nicotiana tabacum cell-wall peptide acts as an in-vitro inhibitor of the cell-wall isoform of acid invertase. Planta, 193, 438-445.

## Patentansprüche

1. Verwendung einer Nukleinsäure, die in einer Pflanzenzelle zur Reduzierung der enzymatischen Aktivität einer Invertase geeignet ist, zur Transformation einer Pflanze, deren aus Samen gezogenen transgenen Nachkommen einSaccharosespeicherorgan ausbilden, bei dem die Saccharosekonzentration gegenüber der Saccharosekonzentration eines nicht veränderten Kontrollsaccharosespeicherorgans des gleichen Genotyps in vergleichbarem Entwicklungsstadium erhöht ist, wobei die Erhöhung der Saccharosekonzentration um X Prozentpunkte zu einem verringerten Saccharosespeicherorganertrag führen kann und die Verringerung des Saccharosespeicherorganertrags maximal 5X Prozent beträgt.

2. Verwendung nach Anspruch 1, bei der der Saccharoseapeicherorganertrag unverändert oder erhöht ist.

3. Verwendung nach Anspruch 1, **dadurch gekennzeichnet, dass** die Saccharosekonzentration des Saccharosespeicherorgans nicht negativ korrellert ist mit dem Saccharosepeicherorganertrag.

4. Verwendung nach Anspruch 1, **dadurch gekennzeichnet, dass** es sich bei der Nukleinsäure um eine homologe oder heterologe Nukleinsäure handelt.

5. Verwendung nach Anspruch 4, **dadurch gekennzeichnet, dass** es sich bei der heterologen Nukleinsäure um eine Nukleinsäure handelt, die für ein zur Reduzierung der enzymatischen Aktivität einer Invertase befähigtes Polypeptid kodiert und die vorzugsweise aus *Nicotiana tabacum* stammt.

6. Verwendung nach Anspruch 4 oder 5, **dadurch gekennzeichnet, dass** die Nukleinsäure eine Sequenz gemäß der SEQ ID NO: 1 oder eine Nukleinsäuresequenz aufweist, die mit einer komplementären Sequenz zu der Sequenz gemäß, der SEQ ID NO:1 oder Abschnitten davon hybridisieren kann.

7. Verwendung nach einem der Ansprüche 1 bis 6, **dadurch gekennzeichnet, dass** es sich bei dem Saccharosespeicherorgan um einen Zuckerrübenkörper oder um einen Zuckerrohrhalm handelt.

8. Verwendung nach Anspruch 5, **dadurch gekennzeichnet, dass** das Polypeptid in einer Zuckerrüben- und Zuckerrohrzelle vakuolär, im Cytosol oder in der Zellwand Iokalisiert ist.

9. Verwendung von Zuckerrüben- oder Zuckerrohrpflanzen mit einer genetischen Ausstattung, die auf die Reduzierung der enzymatischen Aktivität einer Invertase gerichtet ist, zum landwirtschaftlichen Anbau von Zuckerrüben und Zuckerrohr, wobei die genetische Ausstattung die Ausbildung eines Saccharosespeicherorgans ermöglicht, bei dem die Saccharosekonzentration gegenüber der Saccharosekonzentration eines nicht veränderten Kontrollsaccharosespeicherorgans des gleichen Genotyps in vergleichbarem Entwicklungsstadium erhöht ist, wobei die Erhöhung der Saccharosekonzentration um X Prozentpunkte zu einem verringerten Saccharosespeicherorganertrag führt und die Verringerung des Saccharosespeicherorganertrags maximal 5X Prozent beträgt.

10. Verwendung nach Anspruch 9, bei dem der Saccharosespeicherorganertrag unverändert oder erhöht ist.

11. Verwendung nach Anspruch 9, **dadurch gekennzeichnet, dass** die genetische Ausstattung die Ausbildung eines Saccharosespeicherorgans ermöglicht, bei dem die Saccharosekonzentration nicht negativ korreliert ist mit dem Saccharosespeicherorganertrag.

12. Verwendung nach einem der Ansprüche 9 bis 11, **dadurch gekennzeichnet, dass** die Reduzierung der enzymatischen Aktivität einer Invertase mittels einer homologen oder heterologen Nukleinsäure erfolgt.

13. Verwendung nach Anspruch 12, **dadurch gekennzeichnet, dass** es sich bei der heterologen Nukleinsäure um eine Nukleinsäure handelt, die für ein zur Reduzierung der enzymatischen Aktivität einer Invertase befähigtes Polypeptid kodiert und die vorzugsweise aus *Nicotiana tabacum* stammt.

14. Verwendung nach Anspruch 12 oder 13, **dadurch gekennzeichnet, dass** die Nukleinsäure eine Sequenz gemäß der SEQ ID NO: 1 oder eine Nukleinsäuresequenz aufweist, die mit einer komplementären Sequenz zu der Sequenz gemäß der SEQ ID NO: 1 oder Abschnitten davon hybridisieren kann.

15. Verwendung nach Anspruch 12, **dadurch gekennzeichnet, dass** die Nukleinsäure eine Sequenz gemäß der SEQ ID NO: 2 oder eine Nukleinsäuresequenz aufweist, die mit einer komplementären Sequenz zu der Sequenz gemäß der SEQ ID NO: 2 oder Abschnitten davon hybridisieren kann.

## Claims

1. Use of a nucleic acid suitable for reducing the enzymatic activity in a plant cell of an invertase for the transformation of a plant, the transgenic progeny of which, grown from seeds, form a saccharose storage organ, in which the saccharose concentration is increased relative to the saccharose concentration of an unchanged control saccharose storage organ of the same genotype in a comparable development stage, wherein the increase in saccharose concentration by X percentage points can lead to a reduced saccharose storage organ yield and the reduction of the saccharose storage organ yield is a maximum of 5X percent.

2. The use according to Claim 1, in which the saccharose storage organ yield is unchanged or increased.

3. The use according to Claim 1, **characterized in that** the saccharose concentration of the saccharose storage organ is not negatively correlated with the saccharose storage organ yield.

4. The use according to Claim 1, **characterized in that** the nucleic acid is a homologous or heterologous nucleic acid.

5. The use according to Claim 4, **characterized in that** the heterologous nucleic acid is a nucleic acid encoding a polypeptide capable of reducing the enzymatic activity of an invertase and preferably derived from *Nicotiana tabacum.*

6. The use according to Claim 4 or 5, **characterized in that** the nucleic acid includes a sequence according to SEQ ID NO: 1 or a nucleic acid sequence able to hybridize with a sequence complementary to the sequence according to SEQ ID NO: 1 or portions thereof.

7. The use according to one of Claims 1 to 6, **characterized in that** the saccharose storage organ is a sugar beet body or a sugarcane cane.

8. The use according to Claim 5, **characterized in that** the polypeptide is localized in a sugar beet and sugarcane cell in the vacuole, in the cytosol or in the cell wall.

9. Use of sugar beet or sugarcane plants with a genetic makeup which is directed towards the reduction of the enzymatic activity of an invertase, for the agricultural cultivation of sugar beet and sugarcane, wherein the genetic makeup enables the formation of a saccharose storage organ, in which the saccharose concentration is increased relative to the saccharose concentration of an unchanged control saccharose storage organ of the same genotype at a comparable stage of development and wherein the increase in saccharose concentration by X percentage points leads to a reduced saccharose storage organ yield and the reduction of the saccharose storage organ yield is a maximum of 5X percent.

10. The use according to Claim 9, in which the saccharose storage organ yield is unchanged or increased.

11. The use according to Claim 9, **characterized in that** the genetic makeup enables the formation of a saccharose storage organ in which the saccharose concentration is not negatively correlated with the saccharose storage organ yield.

12. The use according to one of Claims 9 to 11, **characterized in that** the reduction of the enzymatic activity of an invertase takes place by means of a homologous or heterologous nucleic acid.

13. The use according to Claim 12, **characterized in that** the heterologous nucleic acid is a nucleic acid encoding a polypeptide capable of reducing the enzymatic activity of an invertase and preferably derived from *Nicotiana tabacum.*

14. The use according to Claim 12 or 13, **characterized in that** the nucleic acid includes a sequence according to SEQ ID NO: 1 or a nucleic acid sequence able to hybridize with a sequence complementary to the sequence according to SEQ ID NO: 1 or portions thereof.

15. The use according to Claim 12, **characterized in that** the nucleic acid includes a sequence according to SEQ ID NO: 2 or a nucleic acid sequence able to hybridize with a sequence complementary to the sequence according to SEQ ID NO: 2 or portions thereof.

## Revendications

1. Utilisation d'un acide nucléique, qui dans une cellule végétale est apte à réduire l'activité enzymatique d'une invertase pour la transformation d'une plante, dont les descendants transgéniques tirés de semences forment un organe de stockage de saccharose pour lequel la concentration en saccharose est élevée par rapport à la concentration en saccharose d'un organe de contrôle de stockage de saccharose non modifié, de génotype identique à un stade de développement comparable, l'augmentation de la concentration en saccharose de la valeur de X pour cent étant susceptible de conduire à un rendement plus faible de l'organe de stockage de saccharose et la réduction du rendement de l'organe de stockage de saccharose étant d'un maximum de 5X pour cent.

2. Utilisation selon la revendication 1, pour laquelle le rendement de l'organe de stockage de saccharose est non modifié ou augmenté.

3. Utilisation selon la revendication 1, **caractérisée en ce que** la concentration en saccharose de l'organe de stockage de saccharose n'est pas en corrélation négative avec le rendement de l'organe de stockage de saccharose.

4. Utilisation selon la revendication 1, **caractérisée en ce que** l'acide nucléique est un acide nucléique homologue ou hétérologue.

5. Utilisation selon la revendication 4, **caractérisée en ce que** l'acide nucléique hétérologue est un acide nucléique qui codifie un polypeptide apte à la réduction de l'activité enzymatique d'une invertase et qui provient de préférence de *Nicotiana tabacum.*

6. Utilisation selon la revendication 4 ou la revendication 5, **caractérisée en ce que** l'acide nucléique présente une séquence selon la SEQ ID NO : 1 ou une séquence d'acide nucléique qui est capable d'hybrider avec une séquence complémentaire à la séquence selon la SEQ ID NO : 1 ou des parties de cette dernière.

7. Utilisation selon l'une quelconque des revendications 1 à 6, **caractérisé en ce que** l'organe de stockage de saccharose est un tubercule de betterave ou une tige de canne à sucre.

8. Utilisation selon la revendication 5, **caractérisée en ce que** le polypeptide dans une cellule de betterave ou de canne à sucre est localisé par vacuoles dans le cytosol ou dans la paroi cellulaire.

9. Utilisation de plants de betteraves ou de canne à sucre avec un équipement génétique qui est axé sur la réduction de l'activité enzymatique d'une invertase pour la culture agricole de betteraves et de la canne à sucre, l'équipement génétique permettant la création d'un organe de stockage de saccharose pour lequel la concentration en saccharose est élevée par rapport à la concentration en saccharose d'un organe de contrôle de stockage de saccharose non modifié, de génotype identique à un stade de développement comparable et l'augmentation de la concentration en saccharose de la valeur de X pour cent étant susceptible de conduire à un rendement plus faible de l'organe de stockage de saccharose et la réduction du rendement de l'organe de stockage de saccharose étant d'un maximum de 5X pour cent.

10. Utilisation selon la revendication 9, pour laquelle le rendement de l'organe de stockage de saccharose est non modifié ou augmenté.

11. Utilisation selon la revendication 9, **caractérisée en ce que** l'équipement génétique permet la création d'un organe de stockage de saccharose pour lequel la concentration en saccharose n'est pas en corrélation négative avec le rendement de l'organe de stockage de saccharose.

12. Utilisation selon l'une quelconque des revendications 9 à 11, **caractérisée en ce que** la réduction de l'activité enzymatique d'une invertase s'effectue au moyen d'un acide nucléique homologue ou hétérologue.

13. Utilisation selon la revendication 12, **caractérisée en ce que** l'acide nucléique hétérologue est un acide nucléique qui codifie un polypeptide apte à la réduction de l'activité enzymatique d'une invertase et qui émane de préférence de *Nicotiana tabacum.*

14. Utilisation selon la revendication 12 ou la revendication 13, **caractérisée en ce que** l'acide nucléique présente une séquence selon la SEQ ID NO : 1 ou une séquence d'acide nucléique qui est capable d'hybrider avec une séquence complémentaire à la séquence selon la SEQ ID NO : 1 ou des parties de cette dernière.

15. Utilisation selon la revendication 12, **caractérisée en ce que** l'acide nucléique présente une séquence selon la SEQ ID NO : 2 ou une séquence d'acide nucléique qui est capable d'hybrider avec une séquence complémentaire à la séquence selon la SEQ ID NO : 2 ou des parties de cette dernière.
